# EUROPEAN PATENT SPECIFICATION

(11) **EP 2 366 028 B1**
(45) Date of publication and mention of the grant of the patent: **10.10.2012**
(21) Application number: 09768220.7
(22) Date of filing: 04.12.2009
(51) Int. Cl.: C12Q 1/18

(54) **METHOD FOR IDENTIFYING INHIBITORS OF LIPOTEICHOIC ACID SYNTHASE**
VERFAHREN ZUR IDENTIFIZIERUNG VON INHIBITOREN VON LIPOTEICHONSÄURE-SYNTHASE
PROCÉDÉ POUR IDENTIFIER DES INHIBITEURS D ACIDE LIPOTÉICHOÏQUE SYNTHASE

(30) Priority: 05.12.2008 GB 0822276
(43) Date of publication of application: 21.09.2011
(73) Proprietor: University of Newcastle Upon Tyne, Newcastle Upon Tyne NE1 7RU (GB)
(72) Inventor: ERRINGTON, Jeffery, Newcastle Upon Tyne NE2 4HH (GB); SCHIRNER, Kathrin, MA 02115 (US)
(74) Representative: Roques, Sarah Elizabeth
(86) International application number: PCT/GB2009/002824
(87) International publication number: WO 2010/064021

(56) References cited:
- WO-A1-2008/131441
- GRUNDLING ANGELIKA ET AL: "Synthesis of glycerol phosphate lipoteichoic acid in Staphylococcus aureus" PROCEEDINGS OF THE NATIONAL ACADEMY OF SCIENCES OF THE UNITED STATES OF AMERICA, vol. 104, no. 20, May 2007 (2007-05), pages 8478-8483, XP007912025 ISSN: 0027-8424 cited in the application
- SCHIRNER KATHRIN ET AL: "Distinct and essential morphogenic functions for wall- and lipo-teichoic acids in Bacillus subtilis" EMBO (EUROPEAN MOLECULAR BIOLOGY ORGANIZATION) JOURNAL, vol. 28, no. 7, April 2009 (2009-04), pages 830-842, XP007912034 ISSN: 0261-4189

### SEQUENCE LISTING

<110> UNIVERSITY OF NEWCASTLE UPON TYNE
<120> METHOD
<130> N105675A SER
<150> GB 0822276.2
   <151> 2008-12-05
<160> 21
<170> Patent In version 3.5
<210> 1
   <211> 2342
   <212> DNA
   <213> Bacillus subtilis
<220>
   <221> CDS
   <222> (196) .. (2192)
<400> 1
   attcctttat ttctagaaag ataccttgtt ttacatttgg taatatcaaa gcgaaacgtt        60
   gattcgacgg cgtttttcgc cactttctcc ctcatacgat tttcactttt ctaatctgct       120
   gattcgtgtt atattggata cgttcgtttt ttctatcgtt tcgctcgaac tggatcggaa       180
   aaaaggagtg taaca atg aaa aca ttt ata aaa gaa aga gga ctg gcc ttc        231
                    Met Lys Thr Phe Ile Lys Glu Arg Gly Leu Ala Phe
                    1               5                   10
   ttc tta att gcg gtc gtc ctg tta tgg atc aaa acg tat gtc ggt tat         279
   Phe Leu Ile Ala Val Val Leu Leu Trp Ile Lys Thr Tyr Val Gly Tyr
           15                  20                  25
   gtc ctg aat ttc aac tta gga ata gac aac acg ata caa aaa ata ttg         327
   Val Leu Asn Phe Asn Leu Gly Ile Asp Asn Thr Ile Gln Lys Ile Leu
       30                  35                  40
   ctt ttt gtg aat cct ctt agc tca agc ttg ttc ttt ctt ggc ttt gga         375
   Leu Phe Val Asn Pro Leu Ser Ser Ser Leu Phe Phe Leu Gly Phe Gly
   45                  50                  55                  60
   ctc ttg ttc aag aaa aaa tta cag cag aca gcc att ata gtg att cat         423
   Leu Leu Phe Lys Lys Lys Leu Gln Gln Thr Ala Ile Ile Val Ile His
                   65                  70                  75
   ttt tta atg tct ttt tta ctg tac gcc aac att gtg tac tac aga ttt         471
   Phe Leu Met Ser Phe Leu Leu Tyr Ala Asn Ile Val Tyr Tyr Arg Phe
               80                  85                  90
   ttc aat gat ttt att aca att ccg gtc att atg cag gct aaa aca aac         519
   Phe Asn Asp Phe Ile Thr Ile Pro Val Ile Met Gln Ala Lys Thr Asn
           95                  100                 105
   ggc ggc caa ctc ggt gac agc gca ttt tcg ctg atg aga ccg act gac         567
   Gly Gly Gln Leu Gly Asp Ser Ala Phe Ser Leu Met Arg Pro Thr Asp
       110                 115                 120
   gcc ttt tac ttt atc gat acg atc atc ctg atc atc ttg gcg atc aaa       615
   Ala Phe Tyr Phe Ile Asp Thr Ile Ile Leu Ile Ile Leu Ala Ile Lys
   125                 130                 135                 140
   gta aac aag cct gcc gaa acg tca agc aaa aaa tcg ttc cga att att       663
   Val Asn Lys Pro Ala Glu Thr Ser Ser Lys Lys Ser Phe Arg Ile Ile
                   145                 150                 155
   ttt gcg tct tca att ctt gtg ttc ttg atc aac ctg gca gtt gcg gaa       711
   Phe Ala Ser Ser Ile Leu Val Phe Leu Ile Asn Leu Ala Val Ala Glu
               160                 165                 170
   tca gac cgt cct gaa ttg ctg aca aga tca ttc gac cgg aac tat ctt       759
   Ser Asp Arg Pro Glu Leu Leu Thr Arg Ser Phe Asp Arg Asn Tyr Leu
           175                 180                 185
   gtg aaa tac ttg gga aca tac aat ttc acg att tat gac gct gta cag       807
   Val Lys Tyr Leu Gly Thr Tyr Asn Phe Thr Ile Tyr Asp Ala Val Gln
       190                 195                 200
   aat atc aag tcc aac agc cag cgc gcg ctt gcc gat tcc agc gac gta       855
   Asn Ile Lys Ser Asn Ser Gln Arg Ala Leu Ala Asp Ser Ser Asp Val
   205                 210                 215                 220
   acg gaa gta gaa aac tac atg aaa gcc aat tac gat gtg ccg aat aac       903
   Thr Glu Val Glu Asn Tyr Met Lys Ala Asn Tyr Asp Val Pro Asn Asn
                   225                 230                 235
   gtg tat ttc ggc aaa gcg gaa gga aaa aac gtc att tac gtt tca ctt       951
   Val Tyr Phe Gly Lys Ala Glu Gly Lys Asn Val Ile Tyr Val Ser Leu
               240                 245                 250
   gaa tct ttg cag tca ttt atc atc gac tat aaa att gac ggc aaa gaa       999
   Glu Ser Leu Gln Ser Phe Ile Ile Asp Tyr Lys Ile Asp Gly Lys Glu
           255                 260                 265
   gtg aca cca ttc tta aat aaa ctg gca cat gat aac gaa acg ttc tac      1047
   Val Thr Pro Phe Leu Asn Lys Leu Ala His Asp Asn Glu Thr Phe Tyr
       270                 275                 280
   ttt gat aac ttt ttc cac caa acg gga caa ggt aaa aca tct gat gct      1095
   Phe Asp Asn Phe Phe His Gln Thr Gly Gln Gly Lys Thr Ser Asp Ala
   285                 290                 295                 300
   gaa ttt atg atg gaa aac tct ctg tac ccg ctg gct caa ggt tca gtt      1143
   Glu Phe Met Met Glu Asn Ser Leu Tyr Pro Leu Ala Gln Gly Ser Val
                   305                 310                 315
   ttc gta aac aaa gcg caa aac acg ctg caa tcc gtt ccg gcg att ctg      1191
   Phe Val Asn Lys Ala Gln Asn Thr Leu Gln Ser Val Pro Ala Ile Leu
               320                 325                 330
   aag tct aag aat tac aca tct gct act ttc cac ggg aac acg cag acg      1239
   Lys Ser Lys Asn Tyr Thr Ser Ala Thr Phe His Gly Asn Thr Gln Thr
           335                 340                 345
   ttc tgg aac cgt aac gaa atg tac aag gcg gaa ggc att gat aaa ttc      1287
   Phe Trp Asn Arg Asn Glu Met Tyr Lys Ala Glu Gly Ile Asp Lys Phe
       350                 355                 360
   ttt gat tct gct tac tat gac atg aac gaa gaa aac acg aaa aac tac      1335
   Phe Asp Ser Ala Tyr Tyr Asp Met Asn Glu Glu Asn Thr Lys Asn Tyr
   365                 370                 375                 380
   ggc atg aaa gac aaa ccg ttc ttc aaa gaa tca atg ccg ctg ctg gaa      1383
   Gly Met Lys Asp Lys Pro Phe Phe Lys Glu Ser Met Pro Leu Leu Glu
                   385                 390                 395
   agc ctg ccg cag ccg ttc tat acg aag ttc att acc ctt tcc aac cac      1431
   Ser Leu Pro Gln Pro Phe Tyr Thr Lys Phe Ile Thr Leu Ser Asn His
               400                 405                 410
   ttc cca ttc gga atg gat gag ggg gat aca gac ttc ccg gct gga gac      1479
   Phe Pro Phe Gly Met Asp Glu Gly Asp Thr Asp Phe Pro Ala Gly Asp
           415                 420                 425
   ttt ggt gac tct gtc gtc gat aac tat ttc cag tca gcc cat tac ctt      1527
   Phe Gly Asp Ser Val Val Asp Asn Tyr Phe Gln Ser Ala His Tyr Leu
       430                 435                 440
   gat cag tcc att gaa caa ttc ttc aat gat ctg aaa aaa gac ggg tta      1575
   Asp Gln Ser Ile Glu Gln Phe Phe Asn Asp Leu Lys Lys Asp Gly Leu
   445                 450                 455                 460
   tat gat aaa tcg att att gtg atg tac gga gac cac tac ggc atc tct      1623
   Tyr Asp Lys Ser Ile Ile Val Met Tyr Gly Asp His Tyr Gly Ile Ser
                   465                 470                 475
   gaa aac cac aat aaa gcg atg gcg aaa gtg ctt ggc aag gat gaa atc      1671
   Glu Asn His Asn Lys Ala Met Ala Lys Val Leu Gly Lys Asp Glu Ile
               480                 485                 490
   act gat tac gac aac gcc cag ctt caa cgg gtg ccg ctc ttt atc cac      1719
   Thr Asp Tyr Asp Asn Ala Gln Leu Gln Arg Val Pro Leu Phe Ile His
           495                 500                 505
   gct gcc ggc gtg aag ggc gag aaa gtt cat aaa tat gcc gga gac gtt      1767
   Ala Ala Gly Val Lys Gly Glu Lys Val His Lys Tyr Ala Gly Asp Val
       510                 515                 520
   gat gtg gct cct acc att ctg cat ctg ctc gga gtg gat acg aag gac      1815
   Asp Val Ala Pro Thr Ile Leu His Leu Leu Gly Val Asp Thr Lys Asp
   525                 530                 535                 540
   tat ctg atg tcc ggt tct gat att tta tcg aaa gaa cac cgt gaa gtg      1863
   Tyr Leu Met Ser Gly Ser Asp Ile Leu Ser Lys Glu His Arg Glu Val
                   545                 550                 555
   att ccg ttc cga aac gga gac ttt att tca ccg aag tac acg aaa ata      1911
   Ile Pro Phe Arg Asn Gly Asp Phe Ile Ser Pro Lys Tyr Thr Lys Ile
               560                 565                 570
   tcc ggt aag tat tac gac acg aaa acc gga aaa gaa ctc gat gaa tcc      1959
   Ser Gly Lys Tyr Tyr Asp Thr Lys Thr Gly Lys Glu Leu Asp Glu Ser
           575                 580                 585
   gaa gtc gac aag tca gaa gac tca ctc gtc aag aag gaa ctt gaa atg      2007
   Glu Val Asp Lys Ser Glu Asp Ser Leu Val Lys Lys Glu Leu Glu Met
       590                 595                 600
   tcc gat aaa atc ata aac gga gac ctg ctg cgg ttc tac gag ccg aaa      2055
   Ser Asp Lys Ile Ile Asn Gly Asp Leu Leu Arg Phe Tyr Glu Pro Lys
   605                 610                 615                 620
   ggt ttt aag aag gtg aat cct tct gat tat gat tac aca aaa cat gac      2103
   Gly Phe Lys Lys Val Asn Pro Ser Asp Tyr Asp Tyr Thr Lys His Asp
                   625                 630                 635
   gaa gat tct tcc gaa acg tca aag gat aac gaa gat aaa taagaaaaag       2152
   Glu Asp Ser Ser Glu Thr Ser Lys Asp Asn Glu Asp Lys
               640                 645
   cggagaggtt gccctctccg ctttttattt tgacagcagc cctcaattcc tcagctgcaa    2212
   attccacatt cgggccaata atgacttgaa ccgattgccc gcccgatttg acaacccctt    2272
   ttgcgcctgc tttctttagc agtgcttcat ccaccaaagc ggtatccttc acagtcagtc    2332
   gcagccttgt                                                           2342
<210> 2
   <211> 649
   <212> PRT
   <213> Bacillus subtilis
<400> 2
   Met Lys Thr Phe Ile Lys Glu Arg Gly Leu Ala Phe Phe Leu Ile Ala
   1               5                   10                  15
   Val Val Leu Leu Trp Ile Lys Thr Tyr Val Gly Tyr Val Leu Asn Phe
               20                  25                  30
   Asn Leu Gly Ile Asp Asn Thr Ile Gln Lys Ile Leu Leu Phe Val Asn
           35                  40                  45
   Pro Leu Ser Ser Ser Leu Phe Phe Leu Gly Phe Gly Leu Leu Phe Lys
       50                  55                  60
   Lys Lys Leu Gln Gln Thr Ala Ile Ile Val Ile His Phe Leu Met Ser
   65                  70                  75                  80
   Phe Leu Leu Tyr Ala Asn Ile Val Tyr Tyr Arg Phe Phe Asn Asp Phe
                   85                  90                  95
   Ile Thr Ile Pro Val Ile Met Gln Ala Lys Thr Asn Gly Gly Gln Leu
               100                 105                 110
   Gly Asp Ser Ala Phe Ser Leu Met Arg Pro Thr Asp Ala Phe Tyr Phe
           115                 120                 125
   Ile Asp Thr Ile Ile Leu Ile Ile Leu Ala Ile Lys Val Asn Lys Pro
       130                 135                 140
   Ala Glu Thr Ser Ser Lys Lys Ser Phe Arg Ile Ile Phe Ala Ser Ser
   145                 150                 155                 160
   Ile Leu Val Phe Leu Ile Asn Leu Ala Val Ala Glu Ser Asp Arg Pro
                   165                 170                 175
   Glu Leu Leu Thr Arg Ser Phe Asp Arg Asn Tyr Leu Val Lys Tyr Leu
               180                 185                 190
   Gly Thr Tyr Asn Phe Thr Ile Tyr Asp Ala Val Gln Asn Ile Lys Ser
           195                 200                 205
   Asn Ser Gln Arg Ala Leu Ala Asp Ser Ser Asp Val Thr Glu Val Glu
       210                 215                 220
   Asn Tyr Met Lys Ala Asn Tyr Asp Val Pro Asn Asn Val Tyr Phe Gly
   225                 230                 235                 240
   Lys Ala Glu Gly Lys Asn Val Ile Tyr Val Ser Leu Glu Ser Leu Gln
                   245                 250                 255
   Ser Phe Ile Ile Asp Tyr Lys Ile Asp Gly Lys Glu Val Thr Pro Phe
               260                 265                 270
   Leu Asn Lys Leu Ala His Asp Asn Glu Thr Phe Tyr Phe Asp Asn Phe
           275                 280                 285
   Phe His Gln Thr Gly Gln Gly Lys Thr Ser Asp Ala Glu Phe Met Met
       290                 295                 300
   Glu Asn Ser Leu Tyr Pro Leu Ala Gln Gly Ser Val Phe Val Asn Lys
   305                 310                 315                 320
   Ala Gln Asn Thr Leu Gln Ser Val Pro Ala Ile Leu Lys Ser Lys Asn
                   325                 330                 335
   Tyr Thr Ser Ala Thr Phe His Gly Asn Thr Gln Thr Phe Trp Asn Arg
               340                 345                 350
   Asn Glu Met Tyr Lys Ala Glu Gly Ile Asp Lys Phe Phe Asp Ser Ala
           355                 360                 365
   Tyr Tyr Asp Met Asn Glu Glu Asn Thr Lys Asn Tyr Gly Met Lys Asp
       370                 375                 380
   Lys Pro Phe Phe Lys Glu Ser Met Pro Leu Leu Glu Ser Leu Pro Gln
   385                 390                 395                 400
   Pro Phe Tyr Thr Lys Phe Ile Thr Leu Ser Asn His Phe Pro Phe Gly
                   405                 410                 415
   Met Asp Glu Gly Asp Thr Asp Phe Pro Ala Gly Asp Phe Gly Asp Ser
               420                 425                 430
   Val Val Asp Asn Tyr Phe Gln Ser Ala His Tyr Leu Asp Gln Ser Ile
           435                 440                 445
   Glu Gln Phe Phe Asn Asp Leu Lys Lys Asp Gly Leu Tyr Asp Lys Ser
       450                 455                 460
   Ile Ile Val Met Tyr Gly Asp His Tyr Gly Ile Ser Glu Asn His Asn
   465                 470                 475                 480
   Lys Ala Met Ala Lys Val Leu Gly Lys Asp Glu Ile Thr Asp Tyr Asp
                   485                 490                 495
   Asn Ala Gln Leu Gln Arg Val Pro Leu Phe Ile His Ala Ala Gly Val
               500                 505                 510
   Lys Gly Glu Lys Val His Lys Tyr Ala Gly Asp Val Asp Val Ala Pro
           515                 520                 525
   Thr Ile Leu His Leu Leu Gly Val Asp Thr Lys Asp Tyr Leu Met Ser
       530                 535                 540
   Gly Ser Asp Ile Leu Ser Lys Glu His Arg Glu Val Ile Pro Phe Arg
   545                 550                 555                 560
   Asn Gly Asp Phe Ile Ser Pro Lys Tyr Thr Lys Ile Ser Gly Lys Tyr
                   565                 570                 575
   Tyr Asp Thr Lys Thr Gly Lys Glu Leu Asp Glu Ser Glu Val Asp Lys
               580                 585                 590
   Ser Glu Asp Ser Leu Val Lys Lys Glu Leu Glu Met Ser Asp Lys Ile
           595                 600                 605
   Ile Asn Gly Asp Leu Leu Arg Phe Tyr Glu Pro Lys Gly Phe Lys Lys
       610                 615                 620
   Val Asn Pro Ser Asp Tyr Asp Tyr Thr Lys His Asp Glu Asp Ser Ser
   625                 630                 635                 640
   Glu Thr Ser Lys Asp Asn Glu Asp Lys
                   645
<210> 3
   <211> 1399
   <212> DNA
   <213> Bacillus subtilis
<220>
   <221> CDS
   <222> (201)..(1199)
<400> 3
   aaattctcga aggagagcct gttcagcaat cgtaatcacc tggcattgcc ttcttgaaat       60
   cgttcataaa acatccgcaa aaatttgtaa agaacttatt gtgcttccaa ctttttttct      120
   atattttatg ataatatata taattagggc acaatgtgga tatttactgt gaaacagatt      180
   ttcaaggagg atataaatag atg ttt gca agg gat att ggt att gac ctc ggt      233
                         Met Phe Ala Arg Asp Ile Gly Ile Asp Leu Gly
                         1               5                   10
   act gca aat gta ctg atc cat gtt aaa ggt aaa gga att gtt ctg aat        281
   Thr Ala Asn Val Leu Ile His Val Lys Gly Lys Gly Ile Val Leu Asn
               15                  20                  25
   gaa cct tcc gtt gtt gca ctt gat aaa aac agc ggc aaa gtg ctg gcg        329
   Glu Pro Ser Val Val Ala Leu Asp Lys Asn Ser Gly Lys Val Leu Ala
           30                  35                  40
   gtt ggc gaa gag gca aga cga atg gtt gga cgt aca cct ggg aat att        377
   Val Gly Glu Glu Ala Arg Arg Met Val Gly Arg Thr Pro Gly Asn Ile
       45                  50                  55
   gtt gcg att cgc ccg ctg aaa gac gga gtt att gct gac ttt gaa gta        425
   Val Ala Ile Arg Pro Leu Lys Asp Gly Val Ile Ala Asp Phe Glu Val
   60                  65                  70                  75
   aca gaa gca atg ctg aaa cat ttt att aac aag ctg aat gta aaa ggc        473
   Thr Glu Ala Met Leu Lys His Phe Ile Asn Lys Leu Asn Val Lys Gly
                   80                  85                  90
   ctg ttc tca aag ccg cgc atg ctc att tgc tgc ccg acg aat att aca        521
   Leu Phe Ser Lys Pro Arg Met Leu Ile Cys Cys Pro Thr Asn Ile Thr
               95                  100                 105
   tcc gtt gag caa aaa gca att aaa gaa gct gca gaa aaa agc ggc ggg        569
   Ser Val Glu Gln Lys Ala Ile Lys Glu Ala Ala Glu Lys Ser Gly Gly
           110                 115                 120
   aaa cat gtg tac ctt gaa gaa gaa cct aaa gtt gcc gct atc ggc gcg        617
   Lys His Val Tyr Leu Glu Glu Glu Pro Lys Val Ala Ala Ile Gly Ala
       125                 130                 135
   ggt atg gaa ata ttc cag cca agc ggt aac atg gtt gta gac atc gga        665
   Gly Met Glu Ile Phe Gln Pro Ser Gly Asn Met Val Val Asp Ile Gly
   140                 145                 150                 155
   ggc ggg acg acg gat atc gcg gtt att tca atg ggc gat att gtc acc        713
   Gly Gly Thr Thr Asp Ile Ala Val Ile Ser Met Gly Asp Ile Val Thr
                   160                 165                 170
   tcc tct tct att aaa atg gct ggg gac aag ttt gac atg gaa atc tta        761
   Ser Ser Ser Ile Lys Met Ala Gly Asp Lys Phe Asp Met Glu Ile Leu
               175                 180                 185
   aat tat atc aaa cgc gag tac aag ctg ctg atc ggc gaa cgt act gcg        809
   Asn Tyr Ile Lys Arg Glu Tyr Lys Leu Leu Ile Gly Glu Arg Thr Ala
           190                 195                 200
   gag gat att aag att aaa gtc gca act gtt ttc cca gac gca cgt cac        857
   Glu Asp Ile Lys Ile Lys Val Ala Thr Val Phe Pro Asp Ala Arg His
       205                 210                 215
   gag gaa att tcc att cgc gga cgg gac atg gtt tcc ggt ctt cca aga        905
   Glu Glu Ile Ser Ile Arg Gly Arg Asp Met Val Ser Gly Leu Pro Arg
   220                 225                 230                 235
   aca att aca gta aac agt aaa gaa gtt gaa gaa gcc ctt cgt gaa tct        953
   Thr Ile Thr Val Asn Ser Lys Glu Val Glu Glu Ala Leu Arg Glu Ser
                   240                 245                 250
   gtc gct gtt att gtt cag gct gca aaa caa gtg ctc gaa aga aca ccg       1001
   Val Ala Val Ile Val Gln Ala Ala Lys Gln Val Leu Glu Arg Thr Pro
               255                 260                 265
   cct gaa ctt tct gct gat att att gac cgc ggc gtt att att acc ggc       1049
   Pro Glu Leu Ser Ala Asp Ile Ile Asp Arg Gly Val Ile Ile Thr Gly
           270                 275                 280
   gga ggc gcg ctc tta aac ggc ctt gac cag ctg ctt gct gaa gag ctg      1097
   Gly Gly Ala Leu Leu Asn Gly Leu Asp Gln Leu Leu Ala Glu Glu Leu
       285                 290                 295
   aag gta ccg gtc ctc gtt gct gaa aat cct atg gat tgc gta gcc atc      1145
   Lys Val Pro Val Leu Val Ala Glu Asn Pro Met Asp Cys Val Ala Ile
   300                 305                 310                 315
   ggc aca ggt gtc atg ctt gat aat atg gac aag ctt cct aaa cgc aaa      1193
   Gly Thr Gly Val Met Leu Asp Asn Met Asp Lys Leu Pro Lys Arg Lys
                   320                 325                 330
   cta agc tgatttcaca aacctcattc tgaaaaagaa tgaggttttt ttatgaaaaa       1249
   Leu Ser
   gccttcacga aaagatgtta aatgacgata ataggataaa atactgagtt tttattatag    1309
   aacgaacgtt cctatatgac aactggaaaa aatgccattt ttagaggtgg gaaatttgtt    1369
   aaaaggatta tatacagcaa catccgcaat                                     1399
<210> 4
   <211> 333
   <212> PRT
   <213> Bacillus subtilis
<400> 4
   Met Phe Ala Arg Asp Ile Gly Ile Asp Leu Gly Thr Ala Asn Val Leu
   1               5                   10                  15
   Ile His Val Lys Gly Lys Gly Ile Val Leu Asn Glu Pro Ser Val Val
               20                  25                  30
   Ala Leu Asp Lys Asn Ser Gly Lys Val Leu Ala Val Gly Glu Glu Ala
           35                  40                  45
   Arg Arg Met Val Gly Arg Thr Pro Gly Asn Ile Val Ala Ile Arg Pro
       50                  55                  60
   Leu Lys Asp Gly Val Ile Ala Asp Phe Glu Val Thr Glu Ala Met Leu
   65                  70                  75                  80
   Lys His Phe Ile Asn Lys Leu Asn Val Lys Gly Leu Phe Ser Lys Pro
                   85                  90                  95
   Arg Met Leu Ile Cys Cys Pro Thr Asn Ile Thr Ser Val Glu Gln Lys
               100                 105                 110
   Ala Ile Lys Glu Ala Ala Glu Lys Ser Gly Gly Lys His Val Tyr Leu
           115                 120                 125
   Glu Glu Glu Pro Lys Val Ala Ala Ile Gly Ala Gly Met Glu Ile Phe
       130                 135                 140
   Gln Pro Ser Gly Asn Met Val Val Asp Ile Gly Gly Gly Thr Thr Asp
   145                 150                 155                 160
   Ile Ala Val Ile Ser Met Gly Asp Ile Val Thr Ser Ser Ser Ile Lys
                   165                 170                 175
   Met Ala Gly Asp Lys Phe Asp Met Glu Ile Leu Asn Tyr Ile Lys Arg
               180                 185                 190
   Glu Tyr Lys Leu Leu Ile Gly Glu Arg Thr Ala Glu Asp Ile Lys Ile
           195                 200                 205
   Lys Val Ala Thr Val Phe Pro Asp Ala Arg His Glu Glu Ile Ser Ile
       210                 215                 220
   Arg Gly Arg Asp Met Val Ser Gly Leu Pro Arg Thr Ile Thr Val Asn
   225                 230                 235                 240
   Ser Lys Glu Val Glu Glu Ala Leu Arg Glu Ser Val Ala Val Ile Val
                   245                 250                 255
   Gln Ala Ala Lys Gln Val Leu Glu Arg Thr Pro Pro Glu Leu Ser Ala
               260                 265                 270
   Asp Ile Ile Asp Arg Gly Val Ile Ile Thr Gly Gly Gly Ala Leu Leu
           275                 280                 285
   Asn Gly Leu Asp Gln Leu Leu Ala Glu Glu Leu Lys Val Pro Val Leu
       290                 295                 300
   Val Ala Glu Asn Pro Met Asp Cys Val Ala Ile Gly Thr Gly Val Met
   305                 310                 315                 320
   Leu Asp Asn Met Asp Lys Leu Pro Lys Arg Lys Leu Ser
                   325                 330
<210> 5
   <211> 23
   <212> DNA
   <213> Artificial Sequence
<220>
   <223> IPCR1 oligonucleotide
<400> 5
   gcttgtaaat tctatcataa ttg 23
<210> 6
   <211> 21
   <212> DNA
   <213> Artificial Sequence
<220>
   <223> IPCR2 oligonucleotide
<400> 6
   agggaatcat ttgaaggttg g 21
<210> 7
   <211> 21
   <212> DNA
   <213> Artificial Sequence
<220>
   <223> IPCR3 oligonucleotide
<400> 7
   gcatttaata ctagcgacgc c 21
<210> 8
   <211> 33
   <212> DNA
   <213> Artificial Sequence
<220>
   <223> mbl-A oligonucleotide
<400> 8
   gctcactctagaccgaggtc aataccaata tcc 33
<210> 9
   <211> 18
   <212> DNA
   <213> Artificial Sequence
<220>
   <223> mbl-B oligonucleotide
<400> 9
   gtgatgaagc gtcctatg 18
<210> 10
   <211> 33
   <212> DNA
   <213> Artificial Sequence
<220>
   <223> mbl-C oligonucleotide
<400> 10
   ctgagcgaat tccgcaaact aagctgattt cac 33
<210> 11
   <211> 19
   <212> DNA
   <213> Artificial Sequence
<220>
   <223> mbl-D oligonucleotide
<400> 11
   cctatatggc ctggaagac 19
<210> 12
   <211> 30
   <212> DNA
   <213> Artificial Sequence
<220>
   <223> mbl-fw oligonucleotide
<400> 12
   ctcgaggatc cacctggcat tgccttcttg 30
<210> 13
   <211> 32
   <212> DNA
   <213> Artificial Sequence
<220>
   <223> mbl-rev oligonucleotide
<400> 13
   catactgaat tccatgacac ctgtgcccga tg 32
<210> 14
   <211> 30
   <212> DNA
   <213> Artificial Sequence
<220>
   <223> yflE-A1 oligonucleotide
<400> 14
   ctagcagcat gcgttcgagc gaaacgatag 30
<210> 15
   <211> 30
   <212> DNA
   <213> Artificial Sequence
<220>
   <223> yflE_A2 oligonucleotide
<400> 15
   gtacggtcta gagttcgagc gaaacgatag 30
<210> 16
   <211> 19
   <212> DNA
   <213> Artificial Sequence
<220>
   <223> yflE-B oligonucleotide
<400> 16
   catcgtgatt ccggcactc 19
<210> 17
   <211> 29
   <212> DNA
   <213> Artificial Sequence
<220>
   <223> yflE-C1 oligonucleotide
<400> 17
   catctaggta ccgagaggtt gccctctcc 29
<210> 18
   <211> 29
   <212> DNA
   <213> Artificial Sequence
<220>
   <223> yflE_C2 oligonucleotide
<400> 18
   ctagctgaat tcgagaggtt gccctctcc 29
<210> 19
   <211> 19
   <212> DNA
   <213> Artificial Sequence
<220>
   <223> yflE-D oligonucleotide
<400> 19
   ctgccgtaat gcatgtcag 19
<210> 20
   <211> 30
   <212> DNA
   <213> Artificial Sequence
<220>
   <223> yflEfw oligonucleotide
<400> 20
   gacagtggat cccactttct ccctcatacg 30
<210> 21
   <211> 30
   <212> DNA
   <213> Artificial Sequence
<220>
   <223> yflErev oligonucleotide
<400> 21
   catccagaat tcgcagctga ggaattgagg 30

## Description

### Field of the Invention

The invention relates to methods or assays to identify agents that can be used as anti-bacterial agents, for example, as antibiotics.

### Background to the Invention

Lipoteichoic acid (LTA) has recently been shown to be essential for *Staphylococcus aureus* viability. An enzyme responsible for assembly of LTA in S. *aureus* has also been described. This enzyme has been named lipoteichoic acid synthase, LtaS. See Gründling and Schneewind, 2007, PNAS 104: 8478-8483.

Homologues of LtaS also exist in other bacteria. For example, *Bacillus* strains express a homolog, previously referred to as *yflE.* Gründling and Schneewind (supra) demonstrate that the *ltaS* homolog of *Bacillus subtilis* can restore LTA synthesis and the growth of *ltaS* mutant staphylococci. *LtaS* inhibition can therefore be used as a target to treat human infections caused by *S*. *aureus* or other bacterial pathogens. Although Gründling and Schneewind (supra) suggest that LtaS might be a useful target for identification of inhibitors which could be used as antibacterial compounds, no specific assay methods are suggested. The assay used by Gründling and Schneewind to find the *ltaS* gene would not be readily adaptable for screening of compounds.

Accordingly, there is a need for an assay to identify inhibitors of LtaS.

Mbl is a bacterial actin homolog that is thought to have a role in cell shape determination by positioning the cell wall synthetic machinery. It is also thought to be involved in the control of other functions including cell plurality and chromosome segregation in various organisms. *Bacillus subtilis* and many other gram positive bacteria have three actin isoforms, one of which is Mbl, which co-localises with two other actin isoforms MreB and MreBH in helical structures that span the length of the cell, close to the inner surface of the cytosplasmic membrane.

Studies carried out with *Bacillus subtilis* have shown that mutants of the *mbl* gene are inviable at normal Mg²⁺ levels. Provision of high concentrations of Mg²⁺, for example, 20 mM rescues such bacillus strains. See Carballido-L6pez et al., 2006, Developmental Cell 11, 399-409.

### Summary of the Invention

The present inventors have identified that transposon mutagenesis can rescue *mbl* mutants. In particular, *Bacillus* strains comprising *mbl* mutations can be subjected to transposon mutagenesis and plated on a low Mg²⁺ medium to identify and select for suppressor mutations which allow growth of the bacteria. Analysis of the transposon mutants identified that inactivation of the *ltaS* gene causes rescue *of mbl* mutants such that such strains can grow at low Mg²⁺ conditions. Accordingly, the present inventors describe assays to identify LtaS inhibitors by identifying substances which are able to rescue growth *of mbl* mutants on low Mg²⁺ medium. These assays are easy and inexpensive cell-based screening methods that allow for screening of a large number of compounds in a straightforward manner.

Thus, in accordance with the first aspects of the present invention, there is provided a method of identifying an inhibitor of LtaS comprising:
(a) providing a gram positive bacteria which comprise a mutation in the *mbl* gene or homologue thereof that discrupts the function of the *mbl* gene or homologue thereof;
(b) culturing the bacteria of (a) in the presence of a test substance under conditions of less than 3 mM magnesium;
(c) monitoring the growth of the bacteria;
wherein growth or more rapid growth of the bacteria compared to growth in the absence of the test substance is indicative that the test substance is an inhibitor of LtaS.

### Description of the Figures

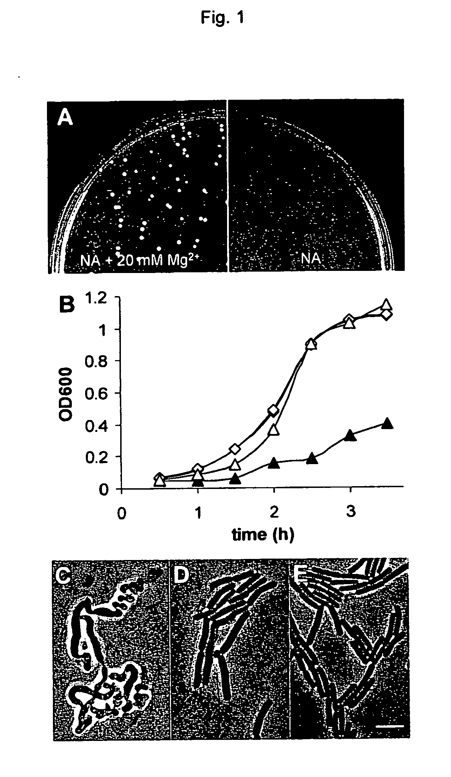

### Fig. 1 - B. subtilis Δmbl is Mg²⁺ dependent

A. Plating efficiency after transformation selecting for deletion of *mbl* with (left) and without (right) addition of 20 mM Mg²⁺. B. Growth curve of *B. subtilis* wild-type (triangles) and *mbl* mutant (squares) at 37°C in PAB medium without (closed symbols) and with (open symbols) addition of 20 mM Mg²⁺. C-E. Morphology (phase-contrast microscopy) of *B. subtilis* Δ*mbl* grown in PAB (C) or in PAB supplemented with 20 mM Mg²⁺ (D) in comparison to a wild-type strain grown in PAB (E). Scale bar 5 µm.

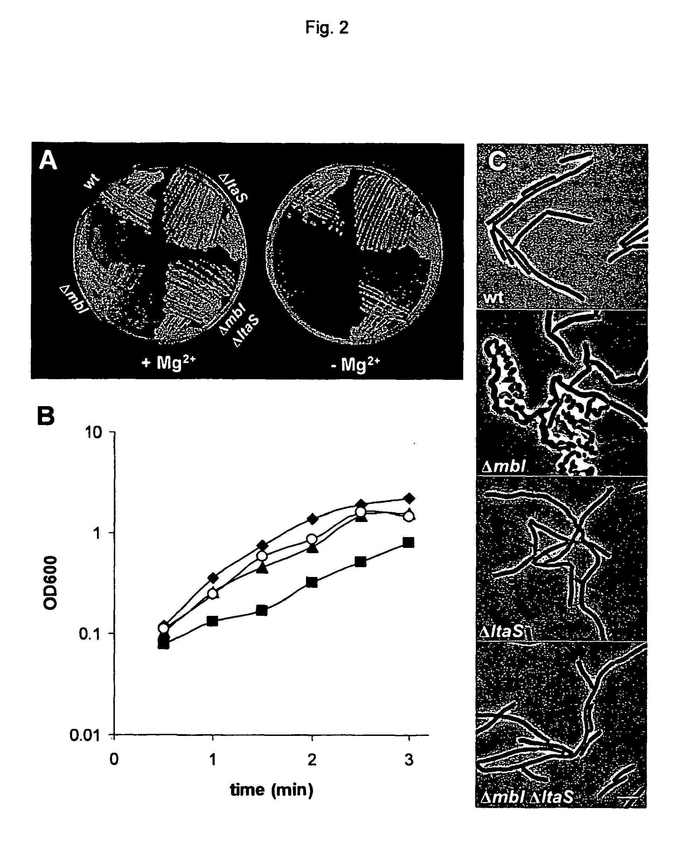

### Fig. 2 - Deletion of ltas suppresses the Mg²⁺ dependency of mbl mutants:

A. Growth of wild-type (168), *mbl* mutant (2505), *ltaS* mutant (4283) and suppressed *mbl* mutant (Δ*mbl* Δ*ltaS,* 4298) on NA plates with (left) or without (right) addition of 20 mM Mg²⁺. B. Growth curves of wild-type (168, ◆), *mbl* mutant (2505, ■), *ltaS* mutant (4283, A) and suppressed *mbl* mutant (Δ*mbl* Δ*ltaS,* 4298, O) in PAB medium at 37°C. C. Phase contrast microscopy of wild-type (168), *mbl* mutant (2505), *ltaS* mutant (4283) and *mbl ltaS* double mutant (4298) grown in PAB medium at 37°C. Scale bar 5 µm.

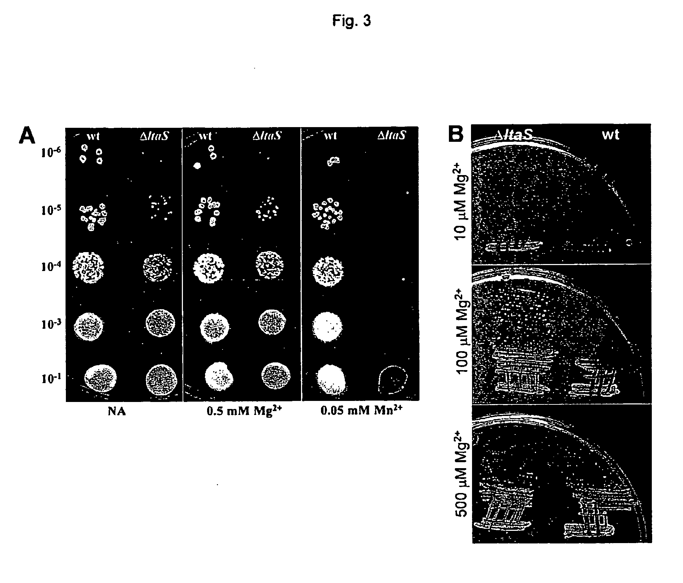

**Fig. 3** **- Effect of metal ion concentration of viability of wild-type and *ltaS* mutants:** A. Growth of wild-type (168) and *ltaS* mutant (strain 4286) at 37°C in on NA plates without additives (left), containing 0.5 mM Mg²⁺ (middle) or with addition of 0.05 mM Mn²⁺ (right). B. Growth of *ltaS* mutant (strain 4283, left) and wild-type (strain 168, right) on minimal medium plates containing 10, 100 and 500 µM Mg²⁺ as indicated.

### Description of the Sequences

Table 4 below sets out the sequences of the genes as discussed in more detail below.
SEQ ID NO: 1 and 2 are the nucleotide and amino acid sequences of *yflE* of *Bacillus subtilis.*
SEQ ID NO: 3 and 4 are the nucleotide and amino acid sequences *of mbl* of *Bacillus subtilis.*

### Detailed Description of the Invention

The present invention provides a method for the identification of an inhibitor of LtaS. LtaS is a lipoteichoic acid synthase. LtaS from *Staphylococcus aureus* has been identified in the prior art, and is described for example in Gründling and Schneewind (supra). Homologues of this gene are also known in other bacterial strains. For example, *Bacillus subtilis* carries a homolog previously identified as *yflE.* The sequence for this gene is set out in SEQ ID NO: 1 and 2.

In accordance with the present invention, a bacterial strain of gram positive bacteria, preferably *Bacillus,* preferably *B. subtilis* is provided. The bacterial strain is selected or modified to include a functional mutation in the *mbl* gene of *B. subtilis* or a homolog thereof of other gram positive bacteria. Mbl is an actin homolog and has been described previously, for example in Abhayawardhane and Stewart, 1995, J. of Bacteriol. 177: 765-773 and Jones et al., Cell 104, 2001, 913-922.

The nucleotide and amino acid sequences for Mbl are set out in Table 4, and labelled as SEQ ID No 3 and 4 respectively. Typically, a homologue *of mbl* from another bacteria is one having more than about 50%, 55% or 65% identity, preferably at least 70%, at least 80%, at least 90% and particularly preferably at least 95%, at least 97% or at least 99% identity, with the amino acid sequence of SEQ ID NO: 4. Such variants may include allelic variants. The identity of variants of SEQ ID NO: 4 may be measured over a region of at least 200, at least 250, at least 300, at least 330 or more contiguous amino acids of the sequence shown in SEQ ID NO: 4 or more preferably over the full length of SEQ ID NO: 4.

Amino acid identity may be calculated using any suitable algorithm. For example the UWGCG Package provides the BESTFIT program which can be used to calculate homology (for example used on its default settings) (Devereux et al. (1984) Nucleic Acids Research 12, 387-395). The PILEUP and BLAST algorithms can be used to calculate homology or line up sequences (such as identifying equivalent or corresponding sequences (typically on their default settings), for example as described in Altschul S. F. (1993) J Mol Evol 36:290-300; Altschul, S. F. et al. (1990) J Mol Biol 215:403-10.

Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al.,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The functional mutation can be any mutation that disrupts the function of the *mbl* gene. Suitable mutations include mutations which disrupt the open reading frame such that a functional Mbl protein cannot be expressed. Alternatively, the mutation may comprise an insertion, for example by transposon mutagenesis to disrupt expression of the gene. In one embodiment, part or all of the *mbl* gene is deleted. Typically, where the gene is deleted, at least 50% of the *mbl* gene is deleted, for example, at least 60%, 70%, 80%, 90% or 95%. Smaller deletions can be included, for example, single base deletions to disrupt the open reading frame or smaller deletions, for example at the N-terminus encoding region such that a functional protein is no longer be expressed. Other mutations that can be incorporated are those mutations causing amino acid substitutions at critical sites in the protein, such as those required for binding of ATP. Any mutation in or around the *mbl* gene that generates a phenotype in which the cells become more dependent on high concentrations of Mg²⁺ in the growth medium can be used.

*mbl* mutants are dependent upon Mg²⁺ for growth. Thus the *mbl* mutants useful in accordance with the present invention are unviable or grow poorly under low Mg²⁺ conditions. Supplementation of the culture medium with Mg²⁺ restores cell growth to such bacterial mutants.

The functional mutations in the *mbl* gene can disrupt the function of the gene such that a functional protein is no longer expressed. Thus, such mutations may affect chromosome segregation or positioning of the cell wall synthetic machinery. Identification of suitable mutants for use in accordance with the present invention can be carried out through a simple analysis of the ability of such mutants to grow under low or normal Mg²⁺. As explained above, *mbl* mutants are dependent on Mg²⁺ for growth. The assay methods in accordance with the present invention use high levels of Mg²⁺, and thus, a suitable mutant for use in accordance with the present invention is one in which a mutation in the *mbl* gene leads to a bacteria which is unviable, or which grows poorly under low Mg²⁺ conditions, for example, in which the doubling time of such a mutant under magnesium concentrations of less than 5 mM is typically greater than 12 hours or greater than 24 hours.

In accordance with the assay methods of the present application, the *mbl* mutant strains are cultured under conditions of low Mg²⁺ in the presence of a test substance. A test substance which acts as an inhibitor of LtaS restores viability of the bacterial strains under such low Mg²⁺ conditions.

Prior to carrying out the assay methods of the present invention, in the presence of a test substance, the *mbl* mutant strains can be grown under conditions of high or supplemented Mg²⁺, such that the bacteria can grow under these conditions. Typically, for bacterial growth of *mbl* mutants, Mg²⁺ is present in the range 1 to 100 mM, preferably 3 mM to 50 mM, preferably 5 mM to 30 mM. For example, growth medium can be supplemented with about 20 mM Mg²⁺. For the purpose of an assay, and completion of the test in a convenient period of time, any medium that supports reasonable growth rate of the *mbl* mutant (e.g. doubling time greater than 120 min at 37°C) can be used.

Typically, a bacterial culture of an *mbl* mutant grown under high Mg²⁺ conditions can be diluted and placed into a sample well. Alternatively, such bacteria can be plated on suitable plates with appropriate growth medium such as agar plates, under low Mg²⁺ conditions. References to low Mg²⁺ conditions relate to magnesium concentrations of less than 3 mM, typically less than 1 mM. Typically, bacteria can be cultured in culture medium which has not been supplemented with Mg²⁺. Thus once the *mbl* mutant bacteria have been diluted or plated out in low Mg²⁺ conditions, their growth will slow or stop.

Low Mg²⁺ conditions can also be identified and defined with respect to bacterial cultures supplemented with 20 mM Mg²⁺. For example, a Mg²⁺ concentration which leads to a growth rate of less than 50%, typically less than 20% or less than 10% of the growth rate of *mbl* mutants grown in 20 mM medium can be used to identify suitable low Mg²⁺ conditions to conduct the assays in accordance with the present invention.

In order to carry out the assays of the present invention, test substances are added to the *mbl* mutant bacteria growing under low Mg²⁺ conditions. For example, test substances can be added to the sample wells or spotted on to plates.

In accordance with the assays of the present invention, bacterial growth of the *mbl* mutants is monitored in the presence of the test substance. Typically, bacteria are grown under usual temperature and time conditions, for example, between 30 and 45°C, typically 37°C. Levels of bacterial growth can be measured at a defined time point, for example, after 2 hours, 4 hours, 6 hours, 8 hours, 12 hours or 24 hours. Alternatively, bacterial growth can be monitored at regular intervals for example every 15 minutes, 30 minutes, hourly, every 2 hours or every 4 hours. Alternatively, bacterial growth can be monitored continuously.

Bacterial growth can be measured by any suitable method. Typically, optical density or a visual assessment of the growth of the bacteria can be carried out. Other suitable methods include use of a dye that generates a colour change during growth (e.g. due to pH change), centrifugation followed by measurement of wet mass, drying followed by measurement of dry mass, chemical determination of a macromolecular component of cells, such as DNA or protein, or counting of cell number microscopically or by an electronic device such as a Coulter counter or flow cytometer, viable cell count by dilution and plating on a suitable growth medium, supplemented with Mg²⁺. Measurement of bacterial growth identifies those mutants whose growth has been rescued despite the low Mg²⁺ conditions. The ability of a test substance to rescue such growth identifies the test substance as an inhibitor of LtaS.

Once an agent has been identified as an inhibitor of LtaS, further studies can be carried out, for example, to assess whether such agent is specific for LtaS. Typically, such test substances can be formulated as pharmaceutical compositions for subsequent administration as antibiotics.

Agents identified in accordance with the present invention can be used as antibiotics against gram positive bacteria, and in particular those which comprise LtaS or a homologue thereof. Such agents are useful in the treatment of *Staphylococcus aureus* infection. Such agents can be used alone or in combination with other antibiotics.

It will be understood that the specific dose level for any particular patient will depend upon a variety of factors including the activity of the specific compound employed, the age, body weight, general health, sex, diet, time of administration, route of administration, rate of excretion, drug combination and the severity of the particular disease undergoing treatment. Optimum dose levels and frequency of dosing will be determined by clinical trial, but an exemplary dosage would be 0.1-1000 mg per day.

The compounds identified in accordance with the present invention may be prepared for administration by any route consistent with their pharmacokinetic properties. The orally administrable compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricant, for example magnesium stearate, talc, polyethylene glycol or silica; disintegrants, for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; non-aqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

The active ingredient may also be administered parenterally in a sterile medium. Depending on the vehicle and concentration used, the drug can either be suspended or dissolved in the vehicle.

The invention is hereinafter described in more detail with reference to the following Examples.

### Example 1

### Lethal effects of mbl deletion can be rescued by high concentrations of magnesium

The actin homologue Mbl has been described as non-essential in *B. subtilis* (Abhayawardhane and Stewart, 1995; Jones *et al.,* 2001), but the former authors had already indicated that *mbl* mutants are slow growing and tend to pick up mutations that enhance growth. The reported Mg²⁺ dependency of both *mreB* (Formstone and Errington, 2005) and (though only at low leves) *mreBH* mutants (Carballido-López *et al.,* 2006) led us to re-construct the *mbl* deletion strain in the presence of 20 mM Mg²⁺. Selecting for transformants under these conditions resulted in a 10-fold increase in plating efficiency giving relatively small but uniformly shaped colonies (Fig 1A). Colonies that were picked continued to grow on Nutrient Agar plates supplemented with Mg²⁺, but failed to grow on unsupplemented plates (Fig 3A). In liquid culture (PAB medium) an elevated magnesium concentration again greatly improved the growth rate (Fig 1B). Microscopic examination of mutant and wild type cells revealed the characteristic twisted and bloated morphology of the mutant in the unsupplemented medium (Fig. 1C). However, in the presence of Mg²⁺, cell morphology was greatly improved (Fig. 1D). Nevertheless, under high Mg²⁺ conditions the *mbl* mutant cells still differed from the wild-type in two ways: first, they were slightly bent and irregularly shaped; second, their average diameter was about 12% greater (Table 1). Wild-type cells had their typical straight rod morphology under both conditions (not shown).

### Screen for magnesium independent suppressor mutants of B. subtilis Δmbl

To gain insight into the function of Mbl we screened for mutants in which the Mg²⁺ dependency of the mutant was suppressed. The plasmid pMarB carrying the *mariner* transposon (Le Breton *et al.,* 2006) was introduced into a freshly constructed Δ*mbl* strain background in the presence of 20 mM Mg²⁺. A library of approximately 60,000 mutants was plated with selection for Mg²⁺ independent growth. Loss of the plasmid pMarB (Erm^{R}), presence of the transposon (Kan^{R}) and disruption of *mbl* (Spc^{R}) were verified by patching on appropriate antibiotic supplemented plates. Ten strong suppressor strains were chosen and checked for linkage of the transposon insertion to the suppression phenotype by three consecutive back-crosses into the Δ*mbl* mutant background. The sites of transposon insertion were determined by sequencing the products of inverse PCR reactions using primers IPCR1-3 (Le Breton *et al.,* 2006).

In two of the ten suppressor strains, the transposon was found to have independently inserted into the *rsgI* gene (previously *ykrI*). RsgI functions as an anti-sigma factor for SigI (Asai *et al.,* 2007). Another hit in the screen was in *yflE* (three independent insertions), encoding a homologue of the lipoteichoic acid synthase LtaS from S. *aureus* (Gründling and Schneewind, 2007). Two independent insertions were found in *ylxA* (synonyms *yllC* or *mraW*) which lies in an operon with *yllB, ftsL,* and *pbpB* and encodes a protein of unknown function. However, *ylxA* deletion proved to be not very potent in suppressing the Mg²⁺ dependency of *B. sublilis* Δ*mbl* (not shown). One transposon insertion each was found in *yaaT* encoding a protein of unknown function involved in the phosphorelay cascade during initiation of sporulation (Hosoya *et al.,* 2002), in the gene for the glutamate transporter GltT (Slotboom *et al.,* 2001; Tolner *et al*., 1992), and in *pnpA* which codes for polynucleotide phosphorylase (Luttinger *et al.,* 1996; Mitra *et al.,* 1996; Wang and Bechhofer, 1996).

### Overlapping but distinct function of the actin homologues in B. subtilis

The finding that mutants *of B. subtilis* actin homologues MreB and MreBH are sensitive to a low Mg²⁺ concentration (Carballido-López *et al.,* 2006; Formstone and Errington, 2005) led us to re-construct the *mbl* mutant in the presence of high concentrations of Mg²⁺. The increase in plating efficiency, uniformity of colony shape, and amelioration of the cell morphology recapitulated the earlier findings made for the *mreB* and *mreBH* mutants. However, the mutants vary in optimal levels of Mg²⁺: the *mreBH* mutant requires only about 100-200 µM Mg²⁺ for viability and the cells display a reduced cell width (Carballido-López *et al*., 2006); the *mreB* mutant has a higher requirement for Mg2⁺ (2.5 mM), and depletion of the cation results in an increase in cell diameter and ultimately lysis (Formstone and Errington, 2005); finally, the newly constructed *mbl* mutant requires addition of about 3 mM Mg²⁺ which is in a similar range of the previously described *mreB* mutant. In unsupplemented medium the strain grows slowly, the cells tend to twist, form chains, swell over their length and are prone to lysis.

In an otherwise wild-type background, the only viable double mutant was Δmbl ΔmreBH, which has a phenotype similar to that of an mbl single mutant. Combinations with ΔmreB were lethal, and depletion of MreB in either mbl or mreBH mutant backgrounds led to a loss of rod-shape and cell death (Defeu Soufo and Graumann, 2006; A. Formstone and J. Errington, unpublished) irrespective of Mg²⁺ levels. Thus, the three MreB-like proteins appear to have overlapping functions, because mreB is essential in strains deleted for any of the other two homologues. However, although the three mutants share certain characteristics like the Mg²⁺ dependency and effects on cell shape, the phenotypic differences between the single mutants show that each has a partially differentiated function.

### Bacterial strains, plasmids and oligonucleotides

*B. subtilis* strains and plasmids used in this study are listed in Table 2, oligonucleotides in Table 3.

### General methods

Liquid cultures *of B. subtilis* strains were grown in Difco Antibiotic Medium 3 (PAB) at 37 or 50°C as indicated. Nutrient agar (Oxoid) plates were used for growth on solid medium. Minimal concentrations of Mg²⁺ required for growth were determined on Nutrient Agar or Modified Salts Medium (Carballido-L6pez *et al.,* 2006). To all media MgSO₄ was added to the indicated final concentration of Mg²⁺. DNA manipulations and *E*. *coli* DH5α transformations were carried out using standard methods (Sambrook, 1989). *B. subtilis* strains were transformed according to the method of Anagnostopoulos and Spizizen (1961) as modified by Jenkinson (1983). Selection for *B. subtilis* transformants was carried out on nutrient agar (Oxoid), supplemented with antibiotics, as required, with: kanamycin (5 mg ml⁻¹) chloramphenicol (5 mg ml⁻¹), erythromycin (1 mg ml⁻¹), lincomycin (25 mg ml⁻¹) and/or spectinomycin (50 mg ml⁻¹). IPTG (1 mM) was added as indicated.

### Screen for Mg²⁺- independent suppressor mutants

Random transposon mutagenesis was performed using the *mariner* based transposon tnYLB-1 as described before (Le Breton *et al*., 2006). In short, the plasmid pMarB was introduced into an *mbl* mutant strain (2505) at 30°C in the presence of high Mg^{z+} concentrations. Individual colonies were picked, grown in LB medium at 37°C for 8 h, and then plated on nutrient agar plates not supplemented with Mg²⁺ but containing kanamycin to select for the transposon insertions creating Mg²⁺ independent strains. Individual colonies were picked and deletion of *mbl* (spc^{r}), integration of the transposon tnYLB-1 (neo^{r}) and loss of the plasmid (erm^{s}) were checked by patching on plates containing the appropriate antibiotic. Linkage between transposon insertion and Mg²⁺ independency was verified by back-crossing chromosomal DNA of single colonies three times into an *mbl* mutant background. Ten strong suppressors were chosen and the site of transposon insertion was determined by inverse PCR amplification and sequencing as described previously (Le Breton *et al.,* 2006).

### Construction of insertional deletion mutants

Chromosomal regions of about 2.5 - 3 kb flanking the gene(s) to be deleted were PCR amplified using primers mbl-A/mbl-B and mbl-C/mbl-D for the *mbl* deletion. These PCR products were then ligated to an antibiotic resistance cassette (*cat* from pCotC; Veening *et al.,* 2006) and then reamplified using the outside primers B + D. Transformation of the resulting PCR products into *B. subtilis* 168 with selection for the adequate antibiotic then gave rise to strains where the target gene is substituted by an antibiotic resistance cassette. Deletion of the gene and insertion of the resistance cassette was verified by PCR.

### Microscopic imaging

For microscopy, cells from an overnight liquid or solid culture were diluted into PAB medium supplemented with 20 mM MgSO₄ when required and grown at 37° or 50°C. Cells were mounted on microscope slides covered with a thin film of 1% agarose in minimal medium (Glaser *et al.,* 1997). Staining of the membrane was achieved by mixing 2 µl of Nile Red (Molecular Probe) solution (12.5 mg ml⁻¹) with 600 µl agarose on the slide. Nucleoids were stained by mixing 8 µl of the cell suspension with 2 µl of DAPI (Sigma) solution (1 mg ml⁻¹ in 50% glycerol) in an Eppendorf cup before mounting the sample on the agarose covered slide. Images were aquired with a 14 Sony CoolSnap HQ cooled CCD camera (Roper Scientific) camera attached to a Zeiss Axiovert M135 microscope or to a Zeiss 15 Axiovert 200M microscope. ImageJ (http://rsb.info.nih.gov/ij/) was used to analyse the images, manipulation was limited to altering brightness and contrast to obtain optimal prints.

**Table 1. Cell dimensions of wild-type and mutant stains**

| Strain | Relevant genotype | Temperature | Mg²⁺ added | Average diameter (± SD) |
|---|---|---|---|---|
| 168 | | 37°C | | 0.92 (0.07) |
| 168 | | 37°C | 20 mM | 0.91 (0.07) |
| 168 | | 50°C | | 0.97 (0.10) |
| 2505 | Δ*mbl* | 37°C | 20 mM | 1.00 (0.09) |
| 2505 | Δ*mbl* | 50°C | | 1.12 (0.12) |

Cultures were grown in PAB medium under the conditions indicated.

**Table 2. Bacterial strains and plasmids**

| Strain/plasmid | Relevant genotype | Reference/construction |
|---|---|---|
| *B. subtilis* | | |
| 168 | *trpC2* | laboratory stock |
| 3728 | *trpC2* Ω*neo3427* Δ*mreB* | Formstone and Errington, 2005 |
| 2505 | *trpC2* Ω*(mbl::spc)* | (Jones *et al.,* 2001) |
| 4261 | *trpC2* Δ*mbl::cat* | this work |
| 4283 | *trpC2* Δ*ltaS::neo* | this work |
| 4284 | *trpC2* Δ*ltaS:spc* | this work |
| 4285 | *trpC2* Δ*ltaS::cat* | this work |
| 4286 | *trpC2* Δ*ltaS::erm* | this work |
| 4298 | *trpC2* Ω*(mbl::spc)*Δ*ltaS::neo* | this work |

| Plasmids | | |
|---|---|---|
| PMarB | *bla erm P_{ctc}-Himar1 kan* (TnYLB-1) | Le Breton *et al*., 2006 |
| pBEST501 | *bla neo* | Itaya *et al*., 1989 |
| pVK71 | *bla neo::spc* | Chary *et al*., 1997 |
| PMUTIN4 | *bla erm Pspac-lacZ lacI* | Vagner *et al.,* 1998 |
| pCotC-GFP | *bla cat P_{cotC}-cotC-gfp* | Veening *et al.,* 2006 |
| pLOSS* | *Bla spc Pspac-mcs P div IVA-lacZ lacI reppLS20* | Claessen *et al.,* 2008 |

**Table 3. Oligonucleotides**

| Name | Sequence | Description |
|---|---|---|
| IPCR1 | GCTTGTAAATTCTATCATAATTG | IPCR amplification |
| IPCR2 | AGGGAATCATTTGAAGGTTGG | IPCR amplification |
| IPCR3 | GCATTTAATACTAGCGACGCC | IPCR DNA sequencing |
| mbl-A | GCTCACTCTAGACCGAGGTCAATACCAATATCC | XbaI |
| mbl-B | GTGATGAAGCGTCCTATG | |
| mbl-C | CTGAGCGAATTCCGCAAACTAAGCTGATTTCAC | EcoRI |
| mbl-D | CCTATATGGCCTGGAAGAC | |
| mbl-fw | CTCGAGGATCCACCTGGCATTGCCTTCTTG | BamHI |
| mbl-rev | CATACTGAATTCCATGACACCTGTGCCCGATG | EcoRI |
| yflE-A1 | CTAGCAGCATGCGTTCGAGCGAAACGATAG | SphI |
| yflE_A2 | GTACGGTCTAGAGTTCGAGCGAAACGATAG | XbaI |
| yflE-B | CATCGTGATTCCGGCACTC | |
| yflE-Cl | CATCTAGGTACCGAGAGGTTGCCCTCTCC | KpnI |
| yflE_C2 | CTAGCTGAATTCGAGAGGTTGCCCTCTCC | EcoRI |
| yflE-D | CTGCCGTAATGCATGTCAG | |
| yflEfw | GACAGTGGATCCCACTTTCTCCCTCATACG | BamHI |
| yflErev | CATCCAGAATTCGCAGCTGAGGAATTGAGG | EcoRI |

### Example 2

### Deletion of the LTA synthase YflE suppresses Mg²⁺ dependency of mbl mutants

We have shown above that *mbl* mutants are not viable at low **[Mg²⁺]** and that mutations suppressing this phenotype can be readily obtained. In a collection of transposon induced suppressed mutants were three strains with insertions in the *yflE* gene. The wild type gene encodes a protein of 649 amino acids with a predicted molecular weight of 74.2 kDa. DNA sequencing showed that each insertion would disrupt the *yflE* open reading frame, after codons 41, 72 and 387, respectively. While the work was in progress, (Gründling and Schneewind, 2007) showed that a closely related gene (79 % identical) in *Staphylococcus aureus* encodes LTA synthase: They also showed that the *yflE* gene of *B. subtilis* could complement the lethal phenotype of *ltaS* in *S. aureus* by restoring LTA synthesis. Therefore, hereafter we rename the *B*. *subtilis yflE* gene as *ltaS.*

We constructed a complete deletion of the *ltaS* gene (strains 4283) and confirmed that the *ltaS mbl* double mutant (strain 4298) is not Mg²⁺ dependent (Fig. 2). Both on plates and in liquid medium (PAB or LB) the double mutant grew without added Mg²⁺ (Fig. 2A and B), although growth was slower than for the wild type culture. Interestingly, deletion of *ltas* also counteracted the typical swelling and twisting *of mbl* mutant cells; instead the double mutant appeared similar to the *ltaS* single mutant (Fig. 2C) (see below).

### Effects of growth conditions and metal ions on ltaS mutants

The *ltaS* mutant also exhibited impaired growth depending on the growth medium. To understand the consequences of loss of LTA synthase activity we characterised the growth of the mutant under a range of conditions. The mutant had a slow growth rate in rich media such as PAB (see below) and it failed to grow at all in CH or S media. Systematic analysis of the effects of components of these media added to PAB showed that the mutant strain was particularly sensitive to elevated Mn²⁺ levels. In the examples shown in Fig. 3A, addition of 0.05 mM MnSO₄ to nutrient agar (NA) abolished growth of the mutant, whereas growth of the wild-type was unaffected. Addition of 0.5 mM Mg²⁺ had no effect on growth of the mutant, showing that the effect was not a general sensitivity to divalent cations. On the other hand we noticed that on minimal media plates with defined Mg²⁺ concentrations the *ltaS* mutant grew at lower Mg²⁺ concentrations than the wild-type strain (Fig. 3B). The lowered requirement for Mg²⁺ might be the reason why a deletion of *ltaS* suppresses the Mg²⁺ dependent phenotype *of mbl* and *mreB* (Formstone and Errington, 2005) mutants. We propose that, consistent with previously suggested functions for LTA in scavenging of Mg²⁺ ions (Neuhaus and Baddiley, 2003), the absence of LTA (synthesized by LtaS) leads to a loss of a buffering zone around the bacterial envelope. As a consequence ions have more immediate access to the cell, leading to a lower requirement for ions with high affinity such as Mg²⁺, which is a co-factor in many bacterial enzymes. At the same time, the toxicity of Mn²⁺ ions increases: these can replace Mg²⁺ because of their similar chemical properties but they do not participate correctly in enzyme function (Cowan, 1995). These results provide direct evidence that LTA has a major role in cell wall physiology and in particular in providing a physicochemical environment that favours the retention of Mg²⁺ over Mn²⁺.

In the process of constructing the deletion strain, we noticed that the *ltaS* mutant was also hyper-sensitive to various antibiotics and lysozyme. As an example, growth of the *ltaS* (strain 4285) mutant was abolished at 0.5 µg/ml kanamycin, a concentration that had no discernible effect on the growth of wild-type cells. In other experiments on solid medium the zone of inhibition of all antibiotics tested (kanamycin, ampicillin, vancomycin, penicillin, spectinomycin, erythromycin, lincomycin, carbenicillin) was wider for the *ltaS* mutant than for the wild-type (not shown). Finally, the mutant also showed increased susceptibility to lysozyme (not shown). The general increase in sensitivity of the mutant to antibiotics and lysozyme is consistent with the notion that LTA also provides a protective layer that restricts the access of many bioactive agents to sensitive sites in the cell envelope.

### Bacterial strains and plasmids

*B. subtilis* strains and plasmids used in this study are listed in Table 2 (supra).

### General methods

Liquid cultures *of B. subtilis* strains were grown in Difco Antibiotic Medium 3 (PAB), CH medium (Nicholson & Setlow, 1990), or S-medium (Karamata & Gross, 1970) at 37°C. Nutrient agar (Oxoid) plates were used for growth on solid medium, Modified Salts Medium plates with defined Mg²⁺ concentrations were prepared as described previously (Carballido-L6pez *et al.,* 2006). The given concentration of Mg²⁺ was achieved by addition of MgSO₄ to the medium. DNA manipulations and *B*. *subtilis* strains were transformed according to the method of Anagnostopoulos and Spizizen (1961) as modified by Jenkinson (1983). Selection for *B. subtilis* transformants was carried out on nutrient agar (Oxoid), supplemented with antibiotics, as required, with: kanamycin (5 mg ml⁻¹) chloramphenicol (5 mg ml⁻¹), erythromycin (1 mg ml⁻¹), lincomycin (25 mg ml⁻¹) and/or spectinomycin (50 mg ml⁻¹). To test the sensitivity to cations, cultures were grown to mid-exponential growth phase in PAB medium, then resuspended in PBS to an OD₆₀₀ of 1.0. 10 µl of dilutions 10⁻¹ to 10⁻⁶ in PBS were spotted on NA plates containing MnSO₄ or MgSO₄ in the concentrations as indicated.

### Screen for Mg²⁺ independent mbl suppressor mutants

Random transposon mutagenesis was performed using the *mariner* based transposon tnYLB-1 as described before (Le Breton *et al.,* 2006). In short, the plasmid pMarB was introduced into an *mbl* mutant strain (2505) at 30°C in the presence of high Mg²⁺ concentrations. Individual colonies were picked, grown in LB medium at 37°C for 8 h, and then plated on nutrient agar plates not supplemented with Mg²⁺ but containing kanamycin to select for the transposon insertions creating Mg²⁺ independent strains. Individual colonies were picked and deletion of *mbl* (spc^{r}), integration of the transposon tnYLB-1 (neo^{r}) and loss of the plasmid (erm^{s}) were checked by patching on plates containing the appropriate antibiotic. Linkage between transposon insertion and Mg²⁺ independency was verified by back-crossing chromosomal DNA of single colonies three times into an *mbl* mutant background. Ten strong suppressors were chosen and the site of transposon insertion was determined by inverse PCR amplification and sequencing as described previously (Le Breton *et al.,* 2006).

### Construction of deletion and depletion strains

Genes were deleted by replacing the coding sequence with antibiotic resistance markers. Therefore, approx. 2500 bp up- and downstream of the target genes were amplified using primers yflE-A/yflE-B and yflE-C/yflE-D for the *yflE* deletion, ligated to the desired resistance cassette and then *B. subtilis* 168 was transformed with the ligation product, transformants were selected on the appropriate antibiotic and verified by PCR. Resistance cassettes were derived by either restriction or PCR amplification from plasmids [*cat* from pCotC (Veening *et al.,* 2006); *erm* from pMUTIN4 (Vagner *et al.,* 1998); *neo* from pBEST501 (Itaya *et al.,* 1989); *spc* from pLOSS* (Claessen *et* a/., 2008)].

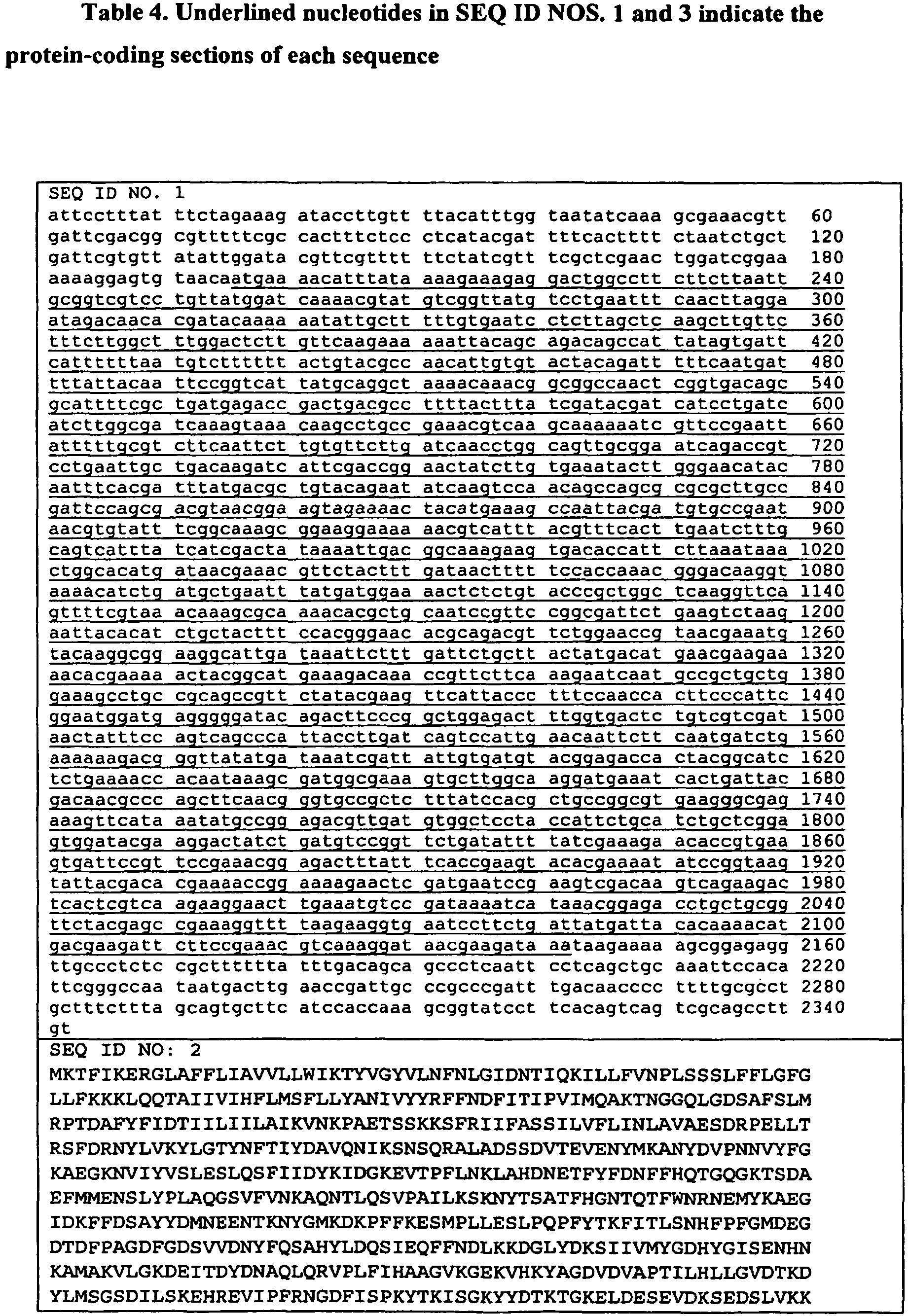

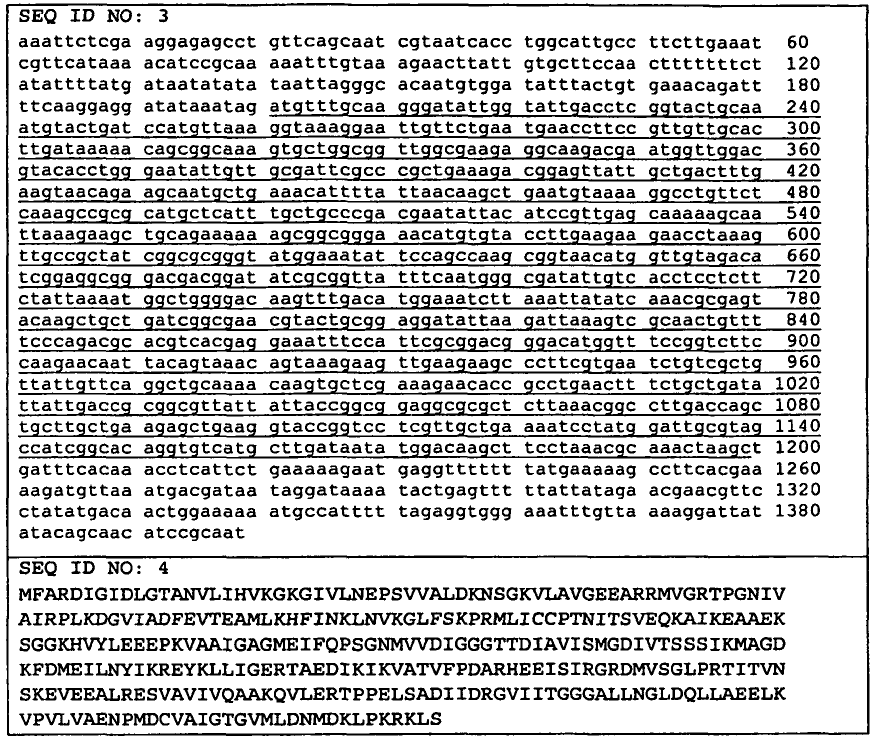

### References

Abhayawardhane, Y., and G.C. Stewart. 1995. Bacillus subtilis possesses a second determinant with extensive sequence similarity to the Escherichia coli mreB morphogene. Journal of Bacteriology 177:765-773.
Anagnostopoulos, C., and J. Spizizen. 1961. Requirements for transformation in Bacillus subtilis. J. Bacteriol. 81:741-746.
Asai, K., T. Ootsuji, K. Obata, T. Matsumoto, Y. Fujita, and Y. Sadaie. 2007. Regulatory role of RsgI in sigl expression in Bacillus subtilis. Microbiology 153:92-101.
Carballido-López, R., A. Formstone, Y. Li, S.D. Ehrlich, P. Noirot, and J. Errington. 2006. Actin homolog MreBH governs cell morphogenesis by localization of the cell wall hydrolase LytE. Developmental Cell 11:399-409.
Chary, V.K., E.I. Amaya, and P.J. Piggot. 1997. Neomycin- and spectinomycin-resistance replacement vectors for Bacillus subtilis. FEMS Microbiology Letters 153:135-139.
Claessen, D.,R. Emmins, L.W. Hamoen, R.A. Daniel, J. Errington, and D.D. Edwards. 2008. Control of the cell elongation-division cycle by shuttling of PBP1 protein in Bacillus subtilis. Molecular Microbiology 68:1029-1046.
Defeu Soufo, H.J., and P.L. Graumann. 2006. Dynamic localization and interaction with other Bacillus subtilis actin-like proteins are important for the function of MreB. Molecular Microbiology 62: 1340-1356.
Formstone, A., and J. Errington. 2005. A magnesium-dependent mreB null mutant: implications for the role of mreB in Bacillus subtilis. Molecular Microbiology 55:1646-1657.
Glaser, P., M.E. Sharpe, B. Raether, M. Perego, K. Ohlsen, and J. Errington. 1997. Dynamic, mitotic-like behavior of a bacterial protein required for accurate chromosome partitioning. Genes Dev. 11:1160-1168.
Gründling, A., and O. Schneewind. 2007. Synthesis of glycerol phosphate lipoteichoic acid in Staphylococcus aureus. Proceedings of the National Academy of Sciences of the United States of America 104:8478-8483.
Hoper, D., U. Volker, and M. Hecker. 2005. Comprehensive characterization of the contribution of individual SigB-dependent general stress genes to stress resistance of Bacillus subtilis. Journal of Bacteriology 187:2810-2826.
Hosoya, S., K. Asai, N. Ogasawara, M. Takeuchi, and T. Sato. 2002. Mutation in yaaT leads to significant inhibition of phosphorelay during sporulation in Bacillus subtilis. Journal of Bacteriology 184:5545-5553.
Itaya, M., K. Kondo, and T. Tanaka. 1989. A neomycin resistance gene cassette selectable in a single copy state in the Bacillus subtilis chromosome. Nucleic Acids Research 17:4410.
Jenkinson, H.F. 1983 Altered arrangement of proteins in the spore coat of a germination mutant of Bacillus subtilis. Journal of General Microbiology 129:1945-1958.
Jones, L.J., R. Carballido-López, and J. Errington. 2001. Control of cell shape in bacteria: helical, actin-like filaments in Bacillus subtilis. Cell 104:913-922.
Le Breton, Y., N.P. Mohapatra, and W.G. Haldenwang. 2006. In Vivo Random Mutagenesis of Bacillus subtilis by Use of TnYLB-1, a mariner-Based Transposon. Appl. Environ. Microbiol. 72:327-333.
Luttinger, A., J. Hahn, and D. Dubnau. 1996. Polynucleotide phosphorylase is necessary for competence development in Bacillus subtilis. Molecular Microbiology 19:343-356**.**
Mitra, S., K. Hue, and D.H. Bechhofer. 1996. In vitro processing activity of Bacillus subtilis polynucleotide phosphorylase. Molecular Microbiology 19:329-342.
Sambrook, J., E.F. Fritsch, T. Maniatis. 1989. Molecular Cloning: A Laboratory Manual. Cold Spring Harbor, NY: Cold Spring Harbor Laboratory Press.
Slotboom, D.J., W.N. Konings, and J.S. Lolkema. 2001. Cysteine-scanning mutagenesis reveals a highly amphipathic, pore-lining membrane-spanning helix in the glutamate transporter GltT. Journal of Biological Chemistry 276:10775-10781.
Tolner, B., B. Poolman, and W.N. Konings. 1992. Characterization and functional expression in Escherichia coli of the sodium/proton/glutamate symport proteins of Bacillus stearothermophilus and Bacillus caldotenax. Molecular Microbiology 6:2845-2856.
Vagner, V., E. Dervyn, and S.D. Ehrlich. 1998. A vector for systematic gene inactivation in Bacillus subtilis. Microbiology 144:3097-3104.
Veening, J.-W., O.P. Kuipers, S. Brul, K.J. Hellingwerf, and R. Kort. 2006. Effects of Phosphorelay Perturbations on Architecture, Sporulation, and Spore Resistance in Biofilms of Bacillus subtilis. J. Bacteriol. 188:3099-3109.
Wang, W., and D.H. Bechhofer. 1996 Properties of a Bacillus subtilis polynucleotide phosphorylase deletion strain. Journal of Bacteriology 178:2375-2382.

## Claims

1. A method of identifying an inhibitor of LtaS comprising:
(a) providing gram positive bacteria in which the bacteria comprise a mutation in the *mbl* gene or homologue thereof that disrupts the function of the *mbl* gene or homologue thereof;
(b) culturing the bacteria of (a) in the presence of a test substance under conditions of less than 3mM magnesium;
(c) monitoring the growth of the bacteria;
wherein growth or more rapid growth of the bacteria compared to growth in the absence of the test substance is indicative that the test substance is an inhibitor of LtaS.

2. A method according to claim 1, wherein the mutation in the *mbl* gene comprises deletion of part or all of the *mbl* gene.

3. A method according to claim 2, wherein the entire *mbl* gene is deleted.

4. A method according to claim 1, wherein the conditions of low magnesium comprise an amount of magnesium such that the bacteria grow at less than 10% of the rate of bacteria having the same *mbl* deletion when grown under conditions of 20mM Mg²⁺.

5. A method according to claim 4, wherein the conditions of low magnesium comprise less than1mM Mg²⁺.

6. A method according to claim 4, wherein the bacteria are cultured in medium unsupplemented by additional Mg²⁺.

7. A method according to claim 1, wherein step (c) comprises monitoring the optical density of the culture to monitor for growth.

8. A method according to claim 7, wherein the method comprises growing an *mbl* mutant bacteria strain in the presence of high Mg²⁺, diluting into low Mg²⁺ medium and transferring to a sample tube, adding a test substance, and monitoring for bacterial growth by monitoring the optical density in the sample well.

9. A method according to claim 1, wherein the bacteria are cultured on an agar plate containing low Mg²⁺ medium, test substance is spotted onto the plate and bacterial growth is detected by visual inspection of the plate.

10. A method according to claim 9, wherein bacteria comprising the *mbl* mutant are cultured in high Mg²⁺ prior to dilution and spreading onto the agar plates.

11. A method according to claim 1, wherein the gram positive bacteria is a bacillus.

12. A method according to claim 11, wherein the bacillus is *B. subtilis.*

## Patentansprüche

1. Verfahren zur Identifizierung eines Inhibitors von LtaS, umfassend:
(a) Bereitstellen von Gram-positiven Bakterien, wobei die Bakterien eine Mutation im *mbl*-Gen oder Homolog davon umfassen, die die Funktion des *mbl*-Gens oder Homologs davon unterbricht;
(b) Kultivieren der Bakterien von (a) in Gegenwart einer Testsubstanz unter Bedingungen von weniger als 3mM Magnesium;
(c) Überwachen des Wachstums der Bakterien;
wobei Wachstum oder schnelleres Wachstum der Bakterien im Vergleich zu Wachstum in Abwesenheit der Testsubstanz anzeigt, dass die die Testsubstanz ein Inhibitor von LtaS ist.

2. Verfahren gemäß Anspruch 1, wobei die Mutation im *mbl*-Gen eine Deletion eines Teils des *mbl*-Gens oder des ganzen *mbl-*Gens umfasst.

3. Verfahren gemäß Anspruch 2, wobei das ganze *mbl*-Gen deletiert wird.

4. Verfahren gemäß Anspruch 1, wobei die Bedingungen von geringer Magnesiumkonzentration eine solche Menge an Magnesium umfassen, dass das Bakterienwachstum weniger als 10% der Rate von Bakterien, die dieselbe *mbl*-Deletion haben, wenn sie unter Bedingungen von 20mM Mg²⁺ kultiviert sind, ist.

5. Verfahren gemäß Anspruch 4, wobei die Bedingungen von geringer Magnesiumkonzentration weniger als 1mm Mg²⁺ umfassen.

6. Verfahren gemäß Anspruch 4, wobei die Bakterien in Medium kultiviert werden, das nicht durch zusätzliches Mg²⁺ supplementiert ist.

7. Verfahren gemäß Anspruch 1, wobei Schritt (c) Überwachen der optischen Dichte der Kultur umfasst, um das Wachstum zu überwachen.

8. Verfahren gemäß Anspruch 7, wobei das Verfahren Wachsen lassen eines *mbl*-mutanten Bakterienstamms in Gegenwart von hoher Mg²⁺-Konzentration, Verdünnen zu Medium mit geringer Mg²⁺-Konzentration und Transferieren in ein Probenröhrchen, Zusetzen einer Testsubstanz und Überwachen bezüglich Bakterienwachstum durch Überwachen der optischen Dichte in dem Probenwell umfasst.

9. Verfahren gemäß Anspruch 1, wobei die Bakterien auf einer Agarplatte, die Medium mit geringer Mg²⁺-Konzentration enthält, kultiviert werden, Testsubstanz auf die Platte aufgetüpfelt wird und bakterielles Wachstum durch visuelle Untersuchung der Platte detektiert wird.

10. Verfahren gemäß Anspruch 9, wobei Bakterien, die die *mbl-*Mutante umfassen, vor Verdünnung und Ausbreitung auf den Agarplatten in hoher Mg²⁺-Konzentration kultiviert werden.

11. Verfahren gemäß Anspruch 1, wobei die Gram-positiven Bakterien Bacillus sind.

12. Verfahren gemäß Anspruch 11, wobei Bacillus *B*. *subtilis* ist.

## Revendications

1. Procédé permettant d'identifier un inhibiteur de LtaS (acide lipotéichoïque synthétase), lequel procédé comporte les étapes suivantes :
a) se procurer des bactéries Gram-positives comportant, au niveau du gène *mbl* ou d'un homologue de celui-ci, une mutation qui empêche le gène *mbl* ou son homologue de remplir sa fonction ;
b) cultiver les bactéries de l'étape (a) en présence d'une substance à tester, dans un milieu contenant du magnésium en une concentration inférieure à 3 mM ;
c) et surveiller la prolifération des bactéries ;
étant entendu que la prolifération des bactéries, ou une prolifération des bactéries plus rapide que leur prolifération en l'absence de la substance testée, indique que la substance testée est un inhibiteur de LtaS.

2. Procédé conforme à la revendication 1, dans lequel la mutation au niveau du gène *mbl* comprend une délétion de tout ou partie du gène *mbl.*

3. Procédé conforme à la revendication 2, dans lequel c'est le gène *mbl* entier qui a fait l'objet de la délétion.

4. Procédé conforme à la revendication 1, dans lequel le milieu à basse concentration de magnésium contient une quantité de magnésium telle que les bactéries prolifèrent à une vitesse qui est inférieure au dixième de la vitesse à laquelle prolifèrent des bactéries porteuses de la même délétion au niveau du gène *mbl* dans un milieu contenant des ions Mg²⁺ en une concentration de 20 mM.

5. Procédé conforme à la revendication 4, dans lequel le milieu à basse concentration de magnésium contient des ions Mg²⁺ en une concentration inférieure à 1 mM.

6. Procédé conforme à la revendication 4, dans lequel on cultive les bactéries dans un milieu qui n'a pas été supplémenté par addition d'ions Mg²⁺.

7. Procédé conforme à la revendication 1, dans lequel l'étape (c) comporte le fait de surveiller la densité optique de la culture, afin de surveiller la prolifération des bactéries.

8. Procédé conforme à la revendication 7, lequel procédé comporte les étapes suivantes : cultiver une souche de bactéries mutantes au niveau de *mbl* dans un milieu à haute concentration d'ions Mg²⁺ ;
diluer la culture de manière à obtenir un milieu à basse concentration d'ions Mg²⁺ et la transférer ensuite dans un puits à échantillon ; y ajouter une substance à tester ; et surveiller la prolifération des bactéries en surveillant la densité optique dans le puits à échantillon.

9. Procédé conforme à la revendication 1, dans lequel on cultive les bactéries sur une plaque de gélose contenant un milieu à basse concentration d'ions Mg²⁺, on dépose la substance à tester en un point sur la plaque, et l'on détecte la prolifération des bactéries par examen visuel de la plaque.

10. Procédé conforme à la revendication 9, dans lequel on cultive des bactéries comprenant des bactéries mutantes au niveau de *mbl* dans un milieu à haute concentration d'ions Mg²⁺, avant de diluer la culture et de la répartir sur des plaques de gélose.

11. Procédé conforme à la revendication 1, dans lequel les bactéries Gram-positives sont des bacilles.

12. Procédé conforme à la revendication 11, dans lequel les bacilles sont de l'espèce *Bacillus subtilis.*
